# EUROPEAN PATENT SPECIFICATION

(11) **EP 1 393 751 B1**
(45) Date of publication and mention of the grant of the patent: **13.06.2007**
(21) Application number: 02019278.7
(22) Date of filing: 28.08.2002
(51) Int. Cl.: A61L 2/24, A61L 2/26, A61L 2/07

(54) **Compact sterilising or desinfection appparatus**
Kompakte Sterilisations- oder Desinfektionsvorrichtung
Dispositif compact pour stériliser ou désinfecter

(43) Date of publication of application: 03.03.2004
(73) Proprietor: Getinge Skärhamn AB, 471 31 Skärhamn (SE)
(72) Inventor: Wanselin, Johan, 47131 Skärhamn (SE)
(74) Representative: Lind, Urban Arvid Oskar

(56) References cited:
- WO-A-02/22180
- WO-A-93/17726
- GB-A- 2 306 328
- US-A- 3 339 785
- US-A- 4 982 655
- US-A- 5 252 303

## Description

### Technical field of the invention

This invention relates to a sterilisation apparatus, comprising a sterilisation unit and a processing unit, wherein said processing unit is arranged to control a sterilisation process within said sterilisation unit.

### Background of the invention

As hygiene is a growing issue around the world, there is a growing need for space and cost efficient sterilisation apparatuses, especially for use in smaller clinics, such as dental practices and district health clinics, often having limited resources, both regarding space and money. Several smaller sterilisation and disinfection devices, specially designed for use in such small clinics are currently on the market, and essentially such a device comprises a sterilisation chamber, in which goods to be sterilised is to be positioned. The device may further comprise a steam generator, connected to the sterilisation chamber and processing means, for controlling the sterilisation process. Such devices has been proven to function rather well, when it comes to sterilisation aspects. However, although such sterilisation devices are much smaller than devices for example used in laboratories and hospitals, a more space-efficient solution is still desirable.

An apparatus according to the preamble of claim 1 is disclosed in DE-A-4037351 showing a radio controlled arrangement with a pocket processing unit.

### Summary of the invention

Hence, an object of this invention is to provide a compact sterilisation apparatus, being easy to manufacture and transport, overcoming at least some of the drawbacks with the prior art mentioned above.

This and other objects are achieved by a sterilisation apparatus according to claim 1. This separation of the sterilisation unit and the processing unit has a plurality of advantages. Only the sterilisation unit has to be positioned so as to be reachable for a user, for example on a bench in a clinic, while the processing unit may be positioned essentially anywhere, where there is sufficient space left, within reasonable reach from the sterilisation unit. Moreover, since most large components, such as a steam generator and a water tank may be positioned in the processing unit, the sterilisation unit may be made essentially smaller. Further, since the above-mentioned components are positioned in the processing unit, additional isolation means (for heat and sound) due to such components, may be excluded from the sterilising unit, thereby making it smaller. Moreover, the inventive separation facilitates easy exchange of one of the units, in case of failure.

Said sterilisation unit comprises a sterilisation chamber, and said processing unit is arranged to be fitted into said sterilisation chamber, for example during transportation of said device, thereby saving transport space and costs.

According to a preferred embodiment of this invention, said sterilisation unit comprises a plurality of modules, such as an sterilisation chamber module and an outer housing module, said modules being easily attached to each other, in order to facilitate exchange of parts but also to facilitate manufacturing.

Moreover, the processing unit may be arranged to be connectable with two or more sterilisation units in order to enable individual control of each of said sterilisation units. Hence, it is possible to save further space and money. It is also possible to allow one sterilisation unit to be driven by two or more processing units.

Suitably, the sterilisation unit comprises a door element, said door element being arranged to be moveable between a first position in which the door is positioned to cover and seal an access opening of said sterilisation chamber and a second position in which the door element is positioned essentially parallel with a side wall of said sterilisation chamber, in order to allow access to the chamber interior. In this way, the sterilisation unit may be made smaller than prior art devices using a door being slidable in one plane. This inventive solution is also advantageous in relation to prior art devices having a door element that opens like a regular door, since the hot side of the door element, after a sterilisation process, will not be accessible for the user and potentially cause burns. Suitably, said door element comprises a turning and sliding shaft extending along a side of said door element, the respective ends of said sliding shaft being arranged between a first and a second guiding rail, so that said door element is turnable about said shaft between said first position and an intermediate position and slidable along said rails between said intermediate position and said second position. Moreover, said guiding rails are positioned on an upper side of said sterilisation chamber, so that the door element in said second position is positioned essentially horizontally above said chamber. Alternatively, said guiding rails are positioned on one of an left or right side of said sterilisation chamber, so that the door element in said second position is position essentially along one of the left or right side of the chamber. Also, the sterilisation apparatus may further comprise an outer housing, wherein the door element, in its second position, is arranged to essentially be positioned in an a space between the chamber wall and the outer housing, so that the surfaces of the door element essentially is unavailable for a user. Also, said door element is further associated with a locking mechanism, for locking the door element in said first position.

Suitably, said processing unit comprises one or more of a processor, a steam generator, a pump, a display, a water supply tank, a plurality of valves, a control card and a water treatment device, being supplied with an arrangement for at least one of detergent and chemical substances., in order to lift the corresponding functions out of the sterilisation unit, in order to miniaturise it.

### Brief description of the drawings

This invention will hereinafter be described in closer detail by means of preferred embodiments thereof, with reference to the accompanying drawings.

Fig 1 is a schematic perspective view of the present invention showing a first and a second sterilisation unit, being connected to a processing unit.

Fig 2 is a schematic view of a sterilisation unit, in which a processing unit is fitted into the sterilisation chamber in order to provide cost-efficient transportation.

Fig 3a-c discloses the door element of the sterilisation unit, in a first, an intermediate and a second position.

### Description of preferred embodiments of this invention

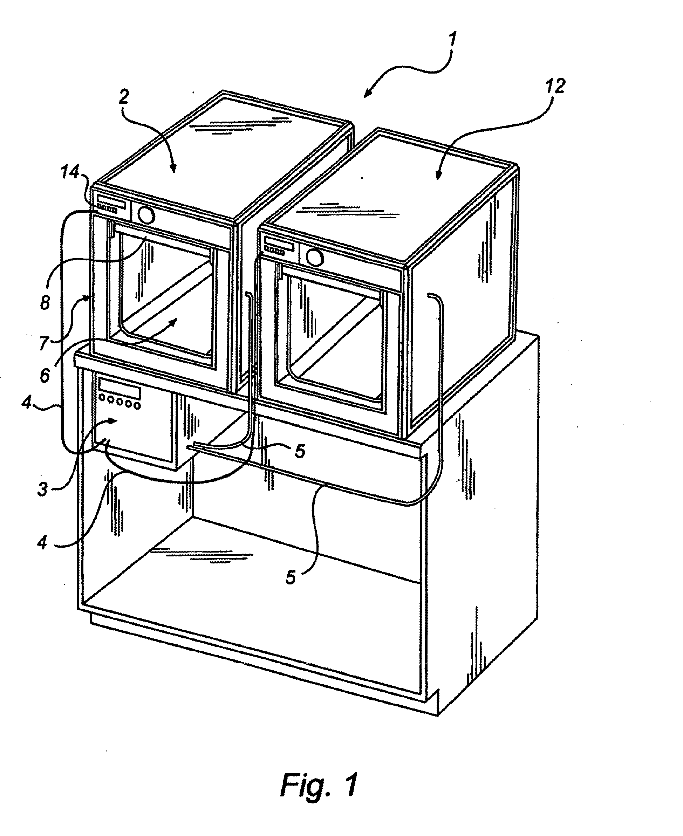

A first embodiment of this invention will hereinafter be described with reference to fig 1. Fig 1 discloses a sterilisation apparatus 1 comprising a sterilisation unit 2 and a processing unit 3. Fig 1 also discloses an optional second sterilisation unit 12, as will be closer described below.

The sterilisation unit 2 and the processing unit 3 are mechanically separated from each other, i.e. are enclosed in separate housings. The sterilisation unit 2 essentially comprises a sterilisation chamber 6, in which items to be sterilised are arranged to be positioned, and an outer sterilisation unit housing 7. The sterilisation chamber 6 may be manufactured from for example a metallic material or a polymeric material. A door element 8 is arranged to alternatively seal or allow access to said chamber 6, and will be described in closer detail below. Between said chamber 6 and said housing 7, heat and sound isolation means (not shown) may be provided. The sterilisation unit 2 may also be equipped with input means 14 for user control of the sterilisation apparatus.

The processing unit 3 comprises in the present case a processor or control card, for controlling a sterilisation process, a water supply or water tank, a steam generator, for generating steam to be used in the sterilisation process, a water treatment device, for treatment of the water to be suitable for the generation of steam (for example by de-ionising), a pump for enabling delivery of fluids, such as steam, water, detergent or chemical substances to said sterilisation chamber, depending on a chosen sterilisation type, a plurality of valves and nozzles for regulating the sterilisation process, and a display means for displaying information regarding the sterilisation cycle. The processing unit may also comprise other components for use in the sterilisation process.

Moreover, the processing unit 3 is connected with the sterilisation unit 2 by means of an information exchange device 4 (such as a communication line) and a medium exchange device 5 (such as steam and/or water piping), for transmitting sterilisation information and sterilisation medium (steam, water, detergent, chemical substances) between the processing unit 3 and the sterilisation unit 2, in accordance with a chosen sterilisation program. The sterilisation program may be chosen by the input means 14 on the sterilisation unit 2, corresponding means on the processing unit 3 or be preprogrammed.

The above separation has a plurality of advantages. First, as may be seen in fig 1, the separation enables that the sterilisation unit 2 may be positioned on the surface of a bench, easily accessible for a user of the sterilisation apparatus, while the processing unit 3 may be positioned at another location, such as under the bench, or in another position where there is available space. Since all processing equipment, such as the steam generator, may be positioned in the processing unit 3, the sterilisation unit 2 may be made comparatively small, since the outer housing essentially only need to enclose the sterilisation chamber 6 itself. Hence, valuable clinic space may be saved. Moreover, since the sterilisation apparatus is subdivided into two separated units, these units may easily be manufactured by different manufacturers, and the sterilisation apparatus may thereafter easily be assembled in the clinic, by connecting the information and medium exchange devices. This total separation also facilitates easy exchange (or maintenance) in the case where one of the units fails or breaks down. A further advantage of this separation is that items such as the steam generator, generating much heat, may be positioned in a unit that is separated from the user, whereby the risk of burns is reduced, while the need of isolation of the sterilisation chamber is also reduced.

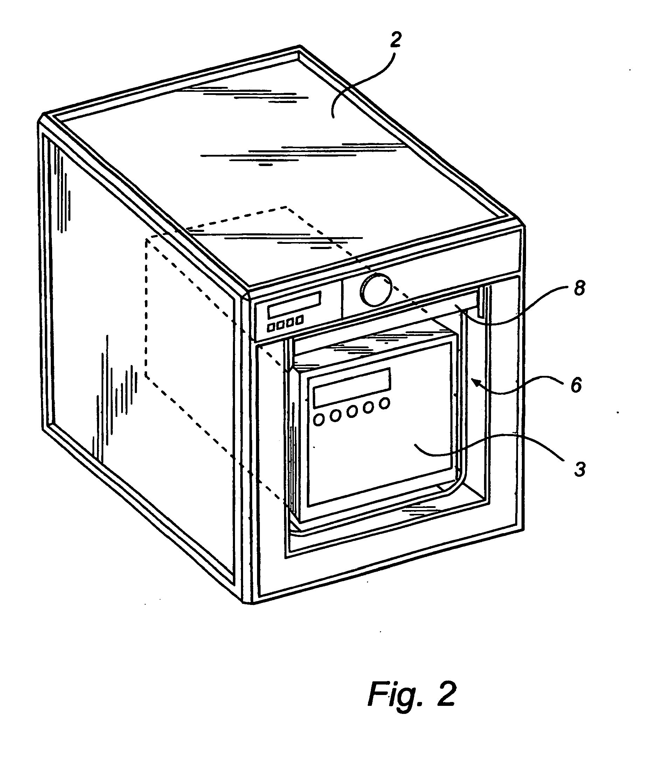

As is seen in fig 2, the processing unit 3 is dimensioned to fit into the sterilisation chamber 6 of the sterilisation unit 2, in order to save valuable space during transportation of the sterilisation apparatus.

As is indicated in fig 1, it is possible to add additional sterilisation units, here a second sterilisation unit 12, to the system, and let a common processing unit 3 control a plurality of sterilisation units. Alternatively, it is possible to use two or more processing units to control one sterilisation unit.

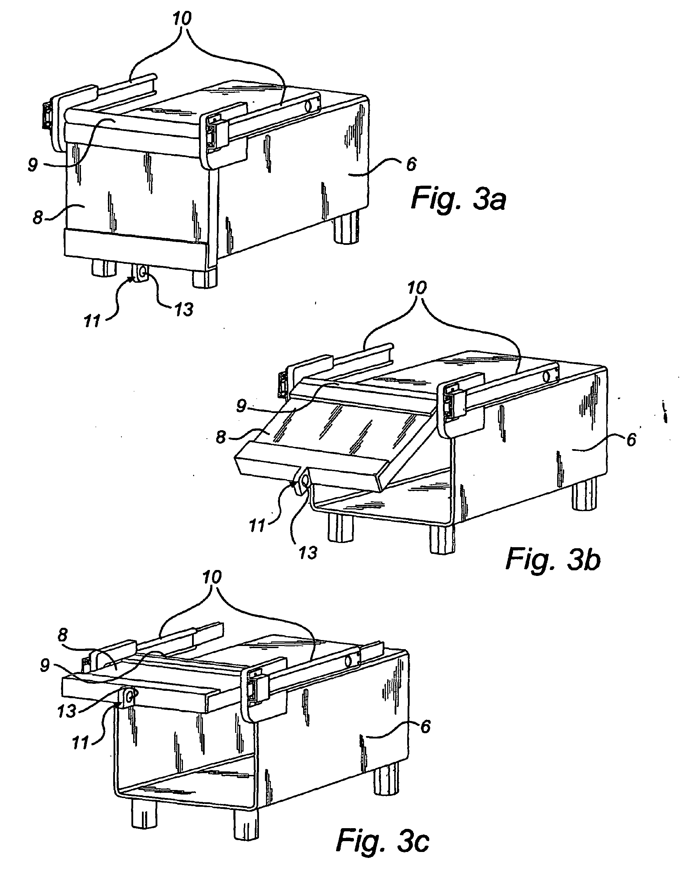

As stated above, the sterilisation unit 2 further comprises a door element 8, said door element being arranged to be moveable between a first position in which the door is positioned to cover and seal an access opening of said sterilisation chamber 6 and a second position in which the door element 8 is positioned essentially parallel with a side wall of said sterilisation chamber 6, in order to allow access to the chamber interior. In both fig 1 and fig 2, the door element is shown in its second position, i.e. allowing access to the interior of the sterilisation chamber 6. The structure of the door element 8 will hereinafter be closer described with reference to fig 3a-3c. The door element structure comprises a door element 8, being of for example a metallic or polymeric material. Depending on the structure, and the pressure environment used in the sterilisation chamber, the door element may or may not comprise reinforcement elements. Along one side of said door element 8, a turning and sliding shaft 9 is arranged. The turning and sliding shaft 9 extend outside the door element 8 on both sides thereof, forming engagement sections 9'. Furthermore, the door element structure comprises a pair of guiding rails 10, being arranged in parallel on top of said sterilisation chamber 6. Said engagement sections 9' of said turning and sliding shaft 9 is arranged to be inserted in and guided by said guiding rails 10, so that said turning and sliding shaft 9 extends between said guiding rails. The distance between said guiding rails 10 is such that the door element 8 may be inserted in-between. Further, the guiding rails 10 extend in front of the sterilisation chamber opening for a distance essentially corresponding with the thickness of the door element 8. Moreover, a spring arrangement may be provided in the guiding rails in order to provide a suitable and flexible movement pattern for the door element.

The movement pattern of the door element 8 will hereinafter be described with reference to fig 3a-3c. In fig 3a, the door element 8b is in a first position, in which the door is positioned to cover and seal an access opening of said sterilisation chamber 6. When the door is in this position, an sterilisation process may be performed within said chamber 6. The door is arranged to be locked in this position by locking means 11, in the present case being constituted by a locking bolt (not shown) being arranged to be inserted into a locking opening 13 arranged on said door element. Alternative locking means are of cause equally applicable. The locking means need however be dimensioned to withstand the operating pressure used within the sterilisation chamber during an sterilisation process. When a sterilisation process has been preformed, and the chamber is to be opened, said locking means 11 is released, and the door element 8 is moved to its second position (the open position) in two steps. First, as is disclosed in fig 3b, the door element 8 is rotated about the turning and sliding shaft 9, with an rotation of approximately 90°, i.e. in this case from a position in which the door element 8 is essentially vertical and covers the access opening of the sterilisation chamber 6, to a position in which the door element 8 is essentially horizontal and parallel with an upper surface of the sterilisation chamber 6. Secondly, as is disclosed in fig 3c, the door element 8 is slidable along the guiding rails 10 to a position in which at least a greater part of the door element 8 is positioned over the sterilisation chamber 6, i.e. the second position, as is shown in fig 1 and 2. It shall be noted that fig 3a-3c discloses only the sterilisation chamber 6 and the door element 8 (and thereto related parts) and do not disclose the outer housing 7, as is the case in fig 1 and 2. It is evident from fig 1 and 2 that the door element, in its second position (open) is positioned in a space between the sterilisation chamber wall and the outer housing 7. This is an advantage since the inner surface of the door element 8, that is facing the interior of the sterilisation chamber 6, may be rather hot (or be covered by hot condensate) after a sterilisation has taken place, and in this way, the inner surface will not be exposed to a user, as is the case with prior art devices having a door element that opens as a regular door. Moreover, this solution is more space efficient than other prior art devices, in which the door element is slidable in a plane perpendicular to the sterilisation chamber opening, since the sterilisation device in this case must be at least twice as broad in the sliding direction as the chamber itself, in order to make room for the door, when the chamber is to be opened. It shall be noted that similar door element arrangements are possible within the scope of this invention, storing the door along the bottom of the sterilisation chamber or along a side of said chamber. However, in the case described above, and showed in fig 3a-3c, only one point of balance is needed in order to provide an even pressure over the door element, which is desirable. Moreover, by constructing the door in the above manner, a space efficient construction is achieved, which is suitable for permanent mounting in a wall, bench or the like.

It shall be noted that a plurality of modifications of the present invention is possible for a man skilled in the art, without departing from the scope, as defined by the appended claims.

For example, it shall be noted that according to a embodiment of the invention, the sterilisation unit comprises a plurality of modules, such as an sterilisation chamber module and an outer housing module, said modules being easily attached to each other. This enables easy exchange of parts, in case of failure. Moreover, it makes it possible to, for example, adapt the design of the chamber for a specific sterilisation purpose, without exchanging the entire sterilisation unit. Moreover, this solution facilitates manufacturing, since certain modules, such as the housing, may be standardised, being adapted to fit with several kinds of chambers.

It shall also be noted that the sterilisation chamber may be provided with two door elements and accompanying chamber openings, suitably arranged on opposite sides of the chamber. This construction enables that goods to be sterilised is loaded into the sterilisation unit via a first chamber opening, while being unloaded through the opposite opening. This may be beneficial in order to increase the total sterilisation time. This construction may also be combined with an automatic loading and unloading system, if desired. Moreover, this construction also enables separation of the sterile side and the non-sterile side of the sterilisation apparatus.

Moreover, if desired, an automatic door element opener may be arranged to control the movement of the door element between the above-described positions. Suitably, such an automatic door element opener may be controlled by the processing unit 3. It shall also be noted that the above-described guiding rails and door element may be alternatively be positioned on either side of the sterilisation chamber, in stead of above the chamber, as disclosed in fig 3a-3c, so that the door element is arranged to be opened to one side. As yet an alternative, the guiding rails may be positioned under the chamber, wherein the door element is arranged to be slid to a position under said chamber.

Hence, by the present invention, a sterilisation apparatus is achieved, which provides a sterilisation unit that is smaller and lighter than a regular sterilisation apparatus. Furthermore it has a low freight volume, and due to the partition in separate units, a sterilisation apparatus may easily be expanded by connecting further sterilising units to an existing processing unit, which is more cost and space efficient than providing an additional prior art device.

## Claims

1. Sterilisation apparatus (1), comprising a sterilisation unit (2) and a processing unit (3), wherein said processing unit (3) is arranged to control a sterilisation process within said sterilisation unit (2), wherein said sterilisation unit (2) comprises a sterilisation chamber (6) for pressurisation, wherein said units (2, 3) are mechanically separated from each other, i.e. are enclosed in separate housings, wherein the units are interconnected by means of at least one information exchange device (4) and wherein said processing unit (3) is dimensioned to be fitted into said sterilisation chamber (6), for example during transportation of said device **characterized in that** said units (2, 3) are additionally interconnected by means of at least one sterilization medium exchange device (5).

2. Sterilisation apparatus according to claim 1, wherein said sterilisation unit (2) comprises a plurality of modules, such as an sterilisation chamber module and an outer housing module, said modules being easily attached to each other.

3. Sterilisation apparatus according to any one of the preceding claims, wherein the processing unit (3) is arranged to be connectable with two or more sterilisation units (2) in order to enable individual control of each of said sterilisation units (2).

4. Sterilisation apparatus according to any one of the preceding claims, wherein the sterilisation unit (2) comprises a door element (8), said door element being arranged to be moveable between a first position in which the door is positioned to cover and seal an access opening of said sterilisation chamber (6) and a second position in which the door element (8) is positioned essentially parallel with a side wall of said sterilisation chamber (6), in order to allow access to the chamber interior.

5. Sterilisation apparatus according to claim 4, wherein said door element (8) comprises a turning and sliding shaft (9) extending along a side of said door element (8), the respective ends of said sliding shaft (9) being arranged between a first and a second guiding rail (10), so that said door element (8) is turnable about said shaft (9) between said first position and an intermediate position and slidable along said rails (10) between said intermediate position and said second position.

6. Sterilisation apparatus according to claim 5, wherein said guiding rails (10) are positioned on an upper side of said sterilisation chamber (6), so that the door element in said second position is positioned essentially horizontally above said chamber (6).

7. Sterilisation apparatus according to claim 6, wherein said guiding rails (10) are positioned on one of an left or right side of said sterilisation chamber (6), so that the door element in said second position is positioned essentially along one of the left .or right side of the chamber .

8. Sterilisation apparatus according to claim 4, 5, 6 or 7 further comprising an outer housing (7), wherein the door element (8), in its second position, is arranged to essentially be positioned in an a space between the chamber wall and the outer housing (7), so that the surfaces of the door element (.8) essentially is unavailable for a user.

9. Sterilisation apparatus according to any one of the claims 4-8, wherein said door element further is associated with a locking mechanism 11, for locking the door element in said first position.

10. Sterilisation apparatus according to any one of the previous claims, wherein said processing unit (3) comprises one or more of a processor, a steam generator, a pump, a display, a water supply tank, a plurality of valves, a control card and a water treatment device, being supplied with an arrangement for at least one of detergent and chemical substances.

11. Sterilisation apparatus according to any one of the previous claims, wherein said sterilisation chamber (6) is manufactured from a metallic or a polymeric material.

12. Sterilisation apparatus according to claim 4-9, wherein the door element (8) being of a metallic or polymeric material.

13. Sterilisation unit, for use in a sterilisation apparatus according to any one of the claims 1-12.

14. Processing unit, for use in a sterilisation apparatus according to any one of the claims 1-12.

## Patentansprüche

1. Sterilisationsvorrichtung (1), die eine Sterilisationseinheit (2) und eine Verarbeitungseinheit (3) umfasst, wobei die Verarbeitungseinheit (3) angeordnet ist, um ein Sterilisationsverfahren innerhalb der Sterilisationseinheit (2) zu steuern, worin die Sterilisationseinheit (2) eine Sterilisationskammer (6) für das Druckfestmachen umfasst, worin die Einheiten (2, 3) mechanisch von einander separiert sind, d.h. in separate Gehäuse eingeschlossen sind, worin die Einheiten mittels mindestens einer Informationsaustauschvorrichtung (4) zusammengeschaltet sind, und worin die Verarbeitungseinheit (3) dimensioniert ist, um in die Sterilisationskammer (6) eingefügt zu werden, zum Beispiel während Transport der Vorrichtung, **dadurch gekennzeichnet , dass** die Einheiten (2, 3) zusätzlich mittels mindestens einer Sterilisationsmittelaustauscheinheit (5) zusammengeschaltet sind.

2. Sterilisationsvorrichtung gemäß Anspruch 1, worin die Sterilisationseinheit (2) eine Vielzahl von Modulen umfasst, wie ein Sterilisationskammermodul und ein äußeres Gehäusemodul, wobei die Module leicht aneinander angeschlossen sind.

3. Sterilisationsvorrichtung gemäß einem der vorhergehenden Ansprüche, worin die Verarbeitungseinheit (3) angeordnet ist, um mit zwei oder mehr Sterilisationseinheiten (2) verknüpfbar zu sein, um individuelle Steuerung von jedem der Sterilisationseinheiten (2) zu ermöglichen.

4. Sterilisationsvorrichtung gemäß einem der vorhergehenden Ansprüche, worin die Sterilisationseinheit (2) ein Türelement (8) umfasst, wobei das Türelement (8) angeordnet ist, um bewegbar zwischen einer ersten Position, in welcher die Tür positioniert ist, um die Zugangsöffnung der Sterilisationskammer (6) zu bedecken und abzudichten, und einer zweiten Position zu sein, in welcher das Türelement (8) im wesentlichen parallel mit der seitlichen Wand der Sterilisationskammer (6) positioniert ist, um Zugang zu dem Kammerinneren zu erlauben.

5. Sterilisationsvorrichtung gemäß Anspruch 4, worin das Türelement (8) einen Dreh- und Gleitschacht (9) umfasst, der sich entlang einer Seite des Türelementes (8) erstreckt, wobei die jeweiligen Enden des Gleitschachtes (9) zwischen einer ersten und einer zweiten Führungsschiene angeordnet (10) sind, so dass das Türelement (8) über den Schacht (9) zwischen der ersten Position und einer Zwischenposition drehbar und verschiebbar entlang der Schienen (10) zwischen der Zwischenposition und der zweiten Position ist.

6. Sterilisationsvorrichtung gemäß Anspruch 5, worin die Führungsschienen (10) auf einer oberen Seite der Sterilisationskammer (6) positioniert sind, so dass das Türelement in der zweiten Position im wesentlichem horizontal über der Kammer (6) positioniert ist.

7. Sterilisationsvorrichtung gemäß Anspruch 6, worin die Führungsschienen (10) auf einer linken oder rechten Seite der Sterilisationskammer (6) positioniert sind, so dass das Türelement in der zweiten Position im wesentlichen entlang einer linken oder rechten Seite der Kammer positioniert ist.

8. Sterilisationsvorrichtung gemäß Anspruch 4, 5, 6 oder 7, die außerdem eine äußeres Gehäuse (7) umfasst, worin das Türelement (8), in seiner zweiten Position, angeordnet ist, um im wesentlichen in einem Raum zwischen der Kammerwand und dem äußeren Gehäuse (7) positioniert zu werden, so dass die Oberflächen des Türelementes (8) im wesentlichen nicht verfügbar für einen Benutzer sind.

9. Sterilisationsvorrichtung gemäß einen der Ansprüche 4-8, worin das Türelement außerdem mit einer Feststellvorrichtung (11) verbunden ist, um das Türelement in der ersten Position festzustellen.

10. Sterilisationsvorrichtung gemäß einem der vorhergehenden Ansprüche, worin Verarbeitungseinheit (3) einen oder mehr von einem Prozessor, einem Dampfgenerator, einer Pumpe, einem Display, einem Wasserbehälter, eine Vielzahl von Ventilen, eine Regelkarte und eine Wasseraufbereitungsvorrichtung umfasst, die beliefert wird mit einer Anordnung für mindestens eines von reinigenden und chemischen Substanzen.

11. Sterilisationsvorrichtung gemäß einem der vorhergehenden Ansprüche, worin die Sterilisationskammer (6) aus einem metallischen oder einem polymeren Material hergestellt ist.

12. Sterilisationsvorrichtung gemäß Anspruch 4-9, worin das Türelement (8) aus einem metallischen oder polymeren Material besteht.

13. Sterilisationsvorrichtung zur Verwendung in einem Sterilisationsgerät gemäß einem der Ansprüche 1-12.

14. Bearbeitungseinheit zur Verwendung in einem Sterilisationsgerät gemäß einem der Ansprüche 1-12.

## Revendications

1. Dispositif de stérilisation (1), comprenant une unité de stérilisation (2) et une unité de traitement (3), dans lequel ladite unité de traitement (3) est conçue pour commander un traitement de stérilisation à l'intérieur de ladite unité de stérilisation (2), et dans lequel ladite unité de stérilisation (2) comprend une chambre de stérilisation (6) pour la mise en pression, dans lequel lesdites unités (2, 3) sont mécaniquement séparées l'une de l'autre, c'est-à-dire sont enfermées dans des boîtiers séparés, dans lequel les unités sont interconnectées au moyen d'au moins un dispositif d'échange d'informations (4) et dans lequel ladite unité de traitement (3) est dimensionnée pour être montée dans ladite chambre de stérilisation (6) par exemple au cours du transport dudit dispositif, **caractérisé en ce que** lesdites unités (2, 3) sont en outre interconnectées au moyen d'au moins un dispositif d'échange de support de stérilisation (5).

2. Dispositif de stérilisation selon la revendication 1, dans lequel ladite unité de stérilisation (2) comprend une pluralité de modules, tel qu'un module de chambre de stérilisation et un module de boîtier extérieur, lesdits modules étant facilement fixés les uns aux autres.

3. Dispositif de stérilisation selon l'une quelconque des revendications précédentes, dans lequel l'unité de traitement (3) est conçue pour pouvoir être connectée à deux unités de stérilisation (2) ou plus afin de permettre une commande individuelle de chacune desdites unités de stérilisation (2).

4. Dispositif de stérilisation selon l'une quelconque des revendications précédentes, dans lequel l'unité de stérilisation (2) comprend un élément de porte (8), ledit élément de porte étant agencé pour être mobile entre une première position, dans laquelle la porte est positionnée pour recouvrir et sceller une ouverture d'accès de ladite chambre de stérilisation (6), et une seconde position, dans laquelle l'élément de porte (8) est positionné de manière essentiellement parallèle à une paroi latérale de ladite chambre de stérilisation (6), afin de permettre un accès à l'intérieur de la chambre.

5. Dispositif de stérilisation selon la revendication 4, dans lequel ledit élément de porte (8) comprend un arbre tournant et coulissant (9) s'étendant le long d'un côté dudit élément de porte (8), les extrémités respectives dudit arbre coulissant (9) étant agencées entre un premier et un second rails de guidage (10), de sorte que ledit élément de porte (8) peut tourner autour dudit arbre (9) entre ladite première position et une position intermédiaire et peut coulisser le long desdits rails (10) entre ladite position intermédiaire et ladite seconde position.

6. Dispositif de stérilisation selon la revendication 5, dans lequel lesdits rails de guidage (10) sont positionnés sur un côté supérieur de ladite chambre de stérilisation (6), de sorte que l'élément de porte dans ladite seconde position est positionné de manière essentiellement horizontale au-dessus de ladite chambre (6).

7. Dispositif de stérilisation selon la revendication 6, dans lequel lesdits rails de guidage (10) sont positionnés sur un côté d'un côté gauche ou droit de ladite chambre de stérilisation (6), de sorte que l'élément de porte dans ladite seconde position est une position essentiellement le long de l'un du côté gauche ou droit de la chambre.

8. Dispositif de stérilisation selon la revendication 4, 5, 6 ou 7 comprenant en outre un boîtier extérieur (7), dans lequel l'élément de porte (8), dans sa seconde position, est agencé pour être essentiellement positionné dans un espace entre la paroi de chambre et le boîtier extérieur (7), de sorte que les surfaces de l'élément de porte (8) sont pratiquement inaccessibles pour un utilisateur.

9. Dispositif de stérilisation selon l'une quelconque des revendications 4 à 8, dans lequel ledit élément de porte est en outre associé à un mécanisme de blocage 11, destiné à bloquer l'élément de porte dans ladite première position.

10. Dispositif de stérilisation selon l'une quelconque des revendications précédentes, dans lequel ladite unité de traitement (3) comprend l'un ou plusieurs d'un processeur, d'un générateur de vapeur, d'une pompe, d'un dispositif d'affichage, d'un réservoir d'alimentation en eau, d'une pluralité de vannes, d'une carte de commande et d'un dispositif de traitement d'eau, qui sont fournis avec un agencement pour au moins une substance parmi des substances détergentes et chimiques.

11. Dispositif de stérilisation selon l'une quelconque des revendications précédentes, dans lequel ladite chambre de stérilisation est fabriquée à partir d'un matériau métallique ou polymère.

12. Dispositif de stérilisation selon les revendications 4 à 9, dans lequel l'élément de porte (8) est d'un matériau métallique ou polymère.

13. Unité de stérilisation, à utiliser dans un dispositif de stérilisation selon l'une quelconque des revendications 1 à 12.

14. Unité de traitement, à utiliser dans un dispositif de stérilisation selon l'une quelconque des revendications 1 à 12.
